(19) Europäisches Patentamt

European Patent Office

Office européen des brevets

(11) **EP 0 608 313 B1**

(12) **EUROPEAN PATENT SPECIFICATION**

(45) Date of publication and mention
of the grant of the patent:
**09.02.2000 Bulletin 2000/06**

(21) Application number: **92921634.9**

(22) Date of filing: **09.10.1992**

(51) Int. Cl.[7]: **A61L 27/00**, A61L 31/00

(86) International application number:
**PCT/US92/08628**

(87) International publication number:
**WO 93/06872 (15.04.1993 Gazette 1993/10)**

(54) **FORMULATIONS OF BLOOD CLOT-POLYMER MATRIX FOR DELIVERY OF OSTEOGENIC PROTEINS**

ZUSAMMENSETZUNGEN, DIE EINE BLUTGERINNSELPOLYMERMATRIX ZUR VERABREICHUNG VON OSTEOGENEN PROTEINEN ENTHALTEN

FORMULATIONS DE CAILLOT DE SANG-MATRICE POLYMERE D'APPORT DE PROTEINES OSTEOGENES

(84) Designated Contracting States:
**AT BE CH DE DK ES FR GB GR IE IT LI LU MC NL SE**

(30) Priority: **11.10.1991 US 776514**

(43) Date of publication of application:
**03.08.1994 Bulletin 1994/31**

(73) Proprietor:
**GENETICS INSTITUTE, INC.
Cambridge, Massachusetts 02140 (US)**

(72) Inventors:
• **RON, Eyal
Lexington, MA 02173 (US)**
• **SCHAUB, Robert, George
Pelham, NH 03076 (US)**
• **TUREK, Thomas, Joseph
Boston, MA 02124 (US)**

(74) Representative:
**Jaenichen, Hans-Rainer, Dr.
Vossius & Partner,
Postfach 86 07 67
81634 München (DE)**

(56) References cited:
**EP-A- 0 341 007        WO-A-85/04185
WO-A-86/00526        WO-A-86/03122
WO-A-90/15586        WO-A-91/05802
WO-A-92/13565        WO-A-93/00049
WO-A-93/00050        GB-A- 2 215 209
US-A- 4 526 909        US-A- 4 563 489
US-A- 4 743 259**

**Description**

BACKGROUND OF THE INVENTION

The subject invention relates to the field of osteogenic proteins and pharmaceutical formulations thereof. More particularly, the subject invention involves pharmaceutical formulations designed to sequester osteogenic protein in-situ for a time sufficient to allow the protein to induce cartilage and/or bone formation.

Osteogenic proteins are those proteins capable of inducing, or assisting in the induction of, cartilage and/or bone formation. Many such osteogenic proteins have in recent years been isolated and characterized, and some have been produced by recombinant methods. For example, so-called bone morphogenic proteins (BMP) have been isolated from demineralized bone tissue (see e.g. Urist US 4,455,256); a number of such BMP proteins have been produced by recombinant techniques (see e.g. Wang et al. US 4,877,864 and Wang et al. US 5,013,549); a family of transforming growth factors (TGF-$\alpha$ and TGF-$\beta$) has been identified as potentially useful in the treatment of bone disease (see e.g. Derynck et al., EP 154,434); a protein designated Vgr-1 has been found to be expressed at high levels in osteogenic cells (see Lyons et al. (1989) Proc. Nat'l. Acad. Sci. USA 86, 4554-4558); and proteins designated OP-1, COP-5 and COP-7 have purportedly shown bone inductive activity (see Oppermann, et al. U.S. 5,001,691).

Various attempts have been made at developing formulations designed to deliver osteogenic proteins to a site where induction of bone formation is desired. For example, certain polymeric matrices such as acrylic ester polymer (Urist, US 4,526,909) and lactic acid polymer (Urist, US 4,563,489) have been utilized, but these formulations do not sequester the osteogenic protein for a time sufficient to optimally induce bone formation, and further have been found to erode too slowly for optimal bone formation.

A biodegradeable matrix of porous particles for delivery of an osteogenic protein designated as OP is disclosed in Kuberasampath, U.S. 5,108,753. While US 5,108,753 discloses that a successful carrier for OP must bind the protein, act as a slow release delivery system, accommodate each step of the cellular response during bone development, and protect the protein from nonspecific proteolysis, no formulations are suggested which contain components that specifically sequester the OP at the site where bone formation is desired.

Okada et al., US 4,652,441, US 4,711,782, US 4,917,893 and US 5,061,492 and Yamamoto et al., US 4,954,298 disclose a prolonged-release microcapsule comprising a polypeptide drug and a drug-retaining substance encapsulated in an inner aqueous layer surrounded by a polymer wall substance in an outer oil layer. Although bone morphogenic protein is listed as a polypeptide capable of such a formation, microencapsulation of osteogenic proteins prevents controlled release of such protein sufficient for optimal bone formation.

Collagen matrices have also been used as delivery vehicles for osteogenic proteins (see e.g. Jeffries, U.S. 4,394,370), but collagen frequently causes undesirable antigenic reactions in patients. Therefore, there remains a need for a pharmaceutical formulation capable of sequestering osteogenic proteins at a site where induction of bone formation is desired for a time sufficient to allow safe, effective induction of such bone formation.

SUMMARY OF THE INVENTION

Applicants have surprisingly discovered that osteogenic proteins can be sequestered at a site where bone inducing activity is desired using blood clot in the absence of an antifibrinolytic agent, provided that a porous particulate polymer matrix is incorporated into the formulation. Therefore, more particularly, the subject invention provides a composition comprising a pharmaceutically acceptable admixture of an osteogenic protein; a porous particulate polymer matrix and an osteogenic protein-sequestering amount of autologous blood clot.

DETAILED DESCRIPTION OF THE INVENTION

The osteogenic proteins useful in the practice of the subject invention are well known to those skilled in the art and include those discussed above. The preferred osteogenic proteins for use herein are those of the BMP class identified as BMP-1 through BMP-8 in US 4,877,864; US 5,013,649; WO 90/11366 published October 4, 1990 and WO 91/18098 published November 28, 1991. The most preferred is BMP-2, the full length cDNA sequence and the ultimate mature protein sequence described in detail in the '649 patent. Of course, combinations of two or more of such osteogenic proteins may be used, as may fragments of such proteins that also exhibit osteogenic activity. Such osteogenic proteins are known to be homodimeric species, but also exhibit activity as mixed heterodimers. Heterodimeric forms of osteogenic proteins may also be used in the practice of the subject invention. Recombinant proteins are preferred over naturally occurring isolated proteins. The amount of osteogenic protein useful herein is that amount effective to stimulate increased osteogenic activity of infiltrating progenitor cells, and will depend upon the size and nature of defect being treated as discussed in more detail below, such amounts being orders of magnitude less than the amount of polymer matrix employed, generally in the range of 1-50 $\mu$g of protein for each 10 mg of polymer matrix employed and more preferably in the range of 0.5-10 $\mu$g protein for each milligram of polymer matrix employed (assuming 0.2 g/cc density).

The osteogenic proteins can be utilized in the form of a pharmaceutically acceptable solution (including reconstitution from a lyophilized form). It is optimal to solubilize the osteogenic protein at concentrations of at least about 1 mg/ml, preferably about 2 mg/ml, so that a pharmaceutically effective amount of protein can be delivered without undue volumes of carrier being necessary. Amino acids having a net positive charge (e.g. net 1+ species such as arginine, histidine, lysine and the ethyl esters of glycine and beta-alanine), preferably a net 2+ charge (e.g. the ethyl ester of histidine, the methyl esters of lysine and arginine, and agmatine), are useful in this regard. Amino acids having a net zero charge are useful in this regard provided that the positive charge of the compound is sufficiently distant (at least 2-3 $CH_2$ units away) from the neutralizing negative charge (e.g. net neutral species such as gamma-amino butyric acid, beta-amino propionic acid, and glycine-glycine dipeptide). Other solubilizing agents useful herein include poly(sorbate), dextran sulfate, guanidine, heparin and sodium chloride. For use in solubilizing BMP-2, the preferred solubilizing agents are arginine and histidine (including esters thereof). Arginine is used in concentrations of 50-600 mM, preferably 300-500mM. Histidine may be added to arginine to solubilize BMP-2, in concentrations of about 1-100 mM, preferably 10-50 mM. When histidine is used alone as a solubilizing agent, it is used in concentrations of about 50-600 mM, preferably 300-500 mM. Various well known methods may be used to compound the osteogenic protein and solubilizing agents for use herein, including but not limited to ultrafiltration, dialysis, gel filtration, and hydrophobic interaction chromatography.

The polymer matrix component useful in the practice of the subject invention is a polymeric material that can be formed into porous particles as described below thereby providing in-situ scaffolding for the osteogenic protein, while having biodegradable properties allowing for replacement by new bone growth. Examples are polymers of amino acids, orthoesters, anhydrides, propylene-co-fumarates, or a polymer of one or more α-hydroxy carboxylic acid monomers, (e.g. α-hydroxy acetic acid (glycolic acid) and/or α-hydroxy propionic acid (lactic acid)). The latter can be employed in its d- or l- form, or as a racemic mixture, the racemic mixture being preferred. When a copolymer of lactic acid and glycolic acid is employed (PLGA), the molar ratio of monomers can range from 1:99 to 99:1 depending upon the desired bioerosion lifetime which in turn depends upon the clinical indication being addressed, as more than 50% of either monomer gives longer bioerosion lifetime (slower biodegradation). The molecular weight of the polymer can range from about 1,000 to 100,000 (relative to polystyrene in $CHCl_3$) with 30,000-50,000 being preferred when a 50:50 copolymer is employed. The higher the molecular weight the slower the biodegradation.

The polymeric matrix component of the subject invention is used in the form of highly porous to hollow (with surface porosity) particles, hereinafter collectively referred to as "porous particles." These porous particles are generally spherical having diameters of 150 to 850 microns (μm), preferably 150-500 μm, most preferably 150-300 μm. This particle size creates sufficient spacing between particles to allow mammalian osteoprogenitor cells to infiltrate and be positively influenced by (evidenced by an increase in osteogenic activity/bone growth rate) the osteogenic protein.

While it has generally been postulated that particles suitable as matrices for delivery of osteogenic proteins should be porous, the extent of porosity necessary to optimally induce bone formation has not previously been studied. The present inventors have discovered that the average surface area per porous particle is critical to optimize bone formation. Specifically, porous particles useful in bone formation according to the present invention should have an average surface area of from about 0.02 to 4 $m^2$/g. The present inventors have further discovered that it is possible to produce porous particles having the desired surface area by introducing a "porosigen" (composition capable of imparting porosity by increasing particle surface area) into the solution used to produce the porous particles. It is also possible to control the bioerosion rate by subjecting the porous particles to sterilizing doses of γ radiation. The higher the γ radiation dose, the faster the bioerosion.

Particles useful herewith have a porosity such that the surface area of the particles is increased about 2-250 fold over the surface area of non-porous particles of comparable size.

A preferred method of production of the porous particles of the invention is, generally speaking, a solvent evaporation process comprising dissolving the polymer (in e.g. $CH_2Cl_2$), and adding a porosigen such as NaCl, mannitol or sucrose in solid and/or liquid form. When porosigen is added in solid form, the matrix-porosigen solution takes the form of a suspension. Another preferred method of production of the porous particles of the invention is a solvent extraction method, wherein the porosigen is added in liquid form with concomitant homogenization. When porosigen is added in liquid form with homogenization, the matrix-porosigen solution takes the form of an emulsion. With either method, the matrix-porosigen emulsion is added to an excess aqueous solution containing surfactant such as poly(vinyl alcohol) with controlled stirring and temperature. The resultant porous particles are hardened by extracting or evaporating residual solvent, and dried. PLGA particles useful in the subject invention made utilizing 50% NaCl as a porosigen have a surface area of between about 0.2 and 0.6 $m^2$/g; and particles made using sucrose as a porosigen have a surface area of between about 0.04 and 0.09 $m^2$/g. PLGA particles of the present invention made using liquid porosigen with homogenization have a surface area of between about 0.02 and 4 $m^2/g$.

The porous nature of the particles of the present invention creates sufficient surface area for protein adsorption and increases biodegradation, the desirable extent of both being dependent upon the clinical indication being addressed. Surface area can be measured by any conventional technique. For example, BET surface area analysis can be employed using a Micromeritics ASAP 2000 system, which measures surface area based upon adsorption and desorption of Krypton gas at the surface and within the pores of the solid sample. The unit calculates and prints out the surface area:

$$\frac{1}{VA[(P_0/P)-1]} = \frac{C-1}{V_mC} (P/P_0) + \frac{1}{V_mC}$$

V = volume absorb at pressure P        $P_0$ = saturation pressure
$P/P_0$ = relative pressure        P = pressure
C = constant        A = gas cross sectional area
Vm = Monolayer Capacity

By plotting

$$\frac{1}{VA((P_0/P)} \text{ vs } P/P_0,$$

the slope being

$$\frac{C-1}{V_mC}$$

and the intercept being

$$\frac{1}{V_mC},$$

the surface area

$$S_t = \frac{V_mNA}{V}$$

where N = Avogadro's number and V = molar volume. The amount of porous particles used to treat a particular defect will, of course, depend upon the size of the defect being treated, and on the effective amount required to adsorb the osteogenic protein.

The protein-sequestering material to be used in the practice of the subject invention is pharmaceutically acceptable human autologous blood. When added to an osteogenic protein/porous particle mixture, the blood clots to form a malleable composite wherein the adsorbed protein is sequestered within the matrix for a time sufficient to allow the protein to increase the otherwise natural rate of osteogenic activity of the infiltrating mammalian progenitor cells. In the absence of such blood clot, osteogenic protein desorbs from the PLGA particles in-situ at a rate such that the osteoinducing effect of the protein is not clinically significant. The ratio of blood to porous particles useful herein is 1:0.5 to 1:10 (v:v), preferably 1:5 (v:v), and more preferably 1:2 (v:v), which ratio represents the amount necessary to prevent desorption from the polymer matrix, yet not so much that the progenitor cells are prevented from infiltrating the matrix, thereby providing the protein the opportunity to assist the osteogenic activity of the progenitor cells. For each 1 ml defect, the amount of blood required will therefore generally be about 0.5-1.0 ml. In cases where large doses of osteogenic protein are employed, clot facilitating agents such as thrombin may be employed to offset the dilution effect of the osteogenic protein. It is preferable to mix the blood component with the solution of osteogenic protein prior to addition of the porous particles.

Additional optional components useful in the practice of the subject application include, e.g. cryogenic protectors such as mannitol, sucrose, lactose, glucose, or glycine (to protect from degradation during lyophilization), antimicrobial preservatives such as methyl and propyl parabens and benzyl alcohol, antioxidants such as EDTA, citrate, and BHT (butylated hydroxytoluene), and surfactants such as poly(sorbates) and poly(oxyethylenes), etc. Of course, the traditional preparation of formulations in pharmaceutically acceptable form (i.e. pyrogen free, appropriate pH and isotonicity, sterility, etc.) is well within the skill in the art and is applicable to the formulations of the subject invention. The osteogenic protein and porous particles of the formulations may be provided to the clinic as a single vial formulation, either as a solution or in lyophilized form, or the formulation may be provided as a multicomponent kit wherein, e.g. the osteogenic protein is provided in one vial and the porous particles are provided in a separate vial. The blood to be used in the formulation is admixed at a time prior to use sufficient to allow clotting, generally 30 to 180 minutes prior to use, taking into account the well-known patient-to-patient variability in clotting time.

The formulations of the subject invention provide malleable implants that allow therapeutically effective amounts of osteoinductive protein to be delivered to an injury site where cartilage and/or bone formation is desired. Such an implant may be used as a substitute for autologous bone graft in fresh and non-union fractures, spinal fusions, and bone defect repair in the orthopaedic field; in cranio/maxillofacial reconstructions; for prosthesis integration, especially as a surface coating to improve fixation of prosthetic implants such as hydroxylapatite coated prostheses; in osteomyelitis for bone regeneration; and in the dental field for erosion of the alveolar ridge and periodontal disease. When used to treat osteomyelitis, the osteogenic protein may be used in combination with porous particles and antibiotics, with the addition of autologous blood as a sequestering agent. The lower viscosity formulations may also be used as a percutaneous injection to accelerate healing of closed fractures. In certain of these uses, the compositions of the subject invention may be used in combination with various bone cements, including erodible bone cements such as poly(propylene-co-fumarate). Also, certain of these uses will utilize bioerodible hardware such as erodible plates, screws, etc. As alluded to above, the dosage regimen will be determined by the clinical indication being addressed, as well as by various patient variables (e.g. weight, age, sex) and clinical presentation (e.g. extent of injury, site of injury, etc.).

EXAMPLE I

PREPARATION OF IMPLANT

A 50:50 random copolymer of lactic acid and glycolic acid (PLGA) having an average molecular weight of 30-40 kD, a number average molecular weight of about 20 kD (by gel permeation chromatography relative to polystyrene standards) and an inherent viscosity of 0.35-0.45 dL/g was dissolved in $CH_2Cl_2$ (15% w/v), and 10 g porosigen (7.5% w/v) was suspended in this solution. The resulting solution was added to an excess poly(vinyl alcohol) aqueous solution (0.1% w/v). After a few hours of stirring under partial vacuum (81.0768 kPa (24 inches Hg)), the particles were hardened in excess cold ethanol (95%) . The resulting particles were washed with water for injection and vacuum dried to give a free-flowing product. BET surface area analysis was performed using a Micrometrics ASAP 2000 system as described above, and the particles had a surface area between about 0.2 and 1.0 $m^2$/g: particles made using sucrose as a porosign had a surface area of between about 0.04 and 0.09 $m^2$/g. The porous particles are sterilized by $\gamma$ irradiation prior to use. For each implant intended for human use, 10 ml of porous particles are placed in a sterile cup.

Lyophilized recombinant human BMP-2 (rhBMP-2) (2 mg) is reconstituted with 1 ml sterile water for injection (WFI). 0.5 ml of the rhBMP-2 solution (1 mg) is drawn into a 3 ml syringe, the needle of which is then replaced with a double female Luer Lok™ connector.

5.5 ml of the patient's venous blood is drawn into a 10 ml syringe (not through a heparinized line), the needle of which is removed and immediately attached to the other side of the Luer Lok™ connector on the 3 ml syringe containing the rhBMP-2 solution. The rhBMP-2 solution is gently transferred into the syringe containing the blood, and the resulting mixture is gently transferred between the syringes four times to assure uniform mixing, ending with the mixture in the 10 ml syringe. The empty 3 ml syringe and Luer Lok connector is removed, and the blood/rhBMP-2 mixture is transferred into the cup containing the porous particles. If necessary, the entire mixture is gently stirred with a stainless steel spatula to achieve uniform consistency. The cup is covered and allowed to sit at room temperature for about one hour. The implant mixture should be used within the following two hours.

When the desired consistency of the malleable implant is obtained, the bony defect is exposed by surgical incision at the site of injury. The implant is applied by the surgeon by shaping the malleable composite of porous particles/rhBMP-2/blood clot to span the bony defect which is desired to be treated. The incision is then closed using conventional methods. Healing is monitored by X-ray analysis of the defect site.

EXAMPLE II

CANINE CALVARIAL DEFECT IMPLANT ANALYSIS

Two male and two female dogs were placed under general anaesthesia and four trephine holes of approximately 12 mm each were made in each skull using an Acra-cut DG II cranial drill, as shown below.

All holes were filled with an implant consisting of rhBMP-2, PLGA porous particles, and autologous blood which had been allowed to clot to form a moldable implant. The dose of rhBMP-2 was approximately 200 μg/ml in each implant. One male and one female animal was sacrificed at 4 weeks and the remaining animals were sacrificed at 8 weeks after surgery.

After sacrifice, the left rostral and right caudal calvarial sites of each animal were used for biomechanical testing, and the right rostral and left caudal calvarial sites were used for histopathology. Biomechanical testing revealed a similar stress resistance in the animals sacrificed at 4 and 8 weeks, which was not statistically significantly different from the untreated control dogs. At both 4 and 8 weeks following surgery, histological examination revealed that bony ingrowth occurred into the implant sites which probably bridged the calvarial defects. Remodeling seemed to be occurring between 4 and 8 weeks with the trabeculae becoming thicker. The porous particulate matrix material was disappearing over the course of the study with little left after 8 weeks.

EXAMPLE III

RAT FEMORAL DEFECT IMPLANT ANALYSIS

A critical-size defect (5 mm) was surgically created in the mid-diaphysis of the left femur of each of 56 male Long Evans retired breeder rats (450-550 grams), by affixing a pre-drilled polyethylene plate to the anterior portion of the femur and excising a segment of bone with a carbide dental drill. A bioerodible implant was prepared by mixing rhBMP-2 (in varying amounts), PLGA porous paticles, and venous rat blood and allowing the blood to clot to form a moldable implant. Eight groups of seven animals each were implanted as follows: 0 µg rhBMP-2; 0.93 µg rhBMP-2; 3.1 µg rhBMP-2; 9.3 µg rhBMP-2; 0 µg rhBMP-2 + 2M ε-amino caproic acid; 0.93 µg rhBMP-2 + 2M ε-amino caproic acid; 3.1 µg rhBMP-2 + 2M ε-amino caproic acid; and 9.3 µg rhBMP-2 + 2M ε-amino caproic acid. (ε-amino caproic acid is a hemostatic agent). The PLGA porous particles used in this study had a surface area of 0.217 m$^2$/g.

Animals were radiographed at Day 0 and Weeks 3, 6, and 9. The animals were sacrificed at week 9, the tissues surrounding the polyethylene plates were removed for histological examination, and the implanted and contralateral non-implanted femurs were harvested. Two femurs in each group were histologically examined, and the remaining femurs were used for biomechanical testing.

Animals receiving 0 µg rhBMP-2 were non-unions at Week 9, and no new bone formation was evident in those groups. Fifty (animals receiving ε-amino caproic acid) to eighty (animals not receiving ε-amino caproic acid) percent of the 0.93 µg dose groups achieved union at Week 9. All animals in the 3.1 µg dose groups achieved union at Week 9. Twelve of thirteen 9.3 µg dose group animals which remained at Week 9 achieved union (one 9.3 µg animal had a plate failure at Week 6). ε-amino caproic acid did not appear to modify the healing response. No bone formation was found in any of the soft tissue surrounding the implants. Some cartilage formation was seen in all implant groups (including the 0 µg rhBMP-2 groups) in the fibrous tissue surrounding the polyethylene plate.

Femurs had a dose-dependent healing response at Week 9. Implants with no rhBMP-2 developed fibrous tissue in the defect area. The 0.93 µg rhBMP-2 dose groups had some fibrous tissue in the defect in addition to areas of poor alignment of the defect site with proximal and distal bone. The 3.1 and 9.3 µg rhBMP-2 implants had little to no fibrous tissue, good alignment of proximal and distal segments and some callus formation. Callus formation was greater in the 9.3 µg groups. Bone marrow appeared normal in all rhBMP-2 dose groups.

No PLGA porous particles were found in any of the rhBMP-2 implant groups. Doses of rhBMP-2 at 3.1 and 9.3 µg/implant gave the most acceptable bone growth.

## Claims

1. A composition capable of inducing cartilage and/or bone formation in a patient comprising a pharmaceutically acceptable admixture of

   (i) an osteogenic protein;
   (ii) a porous particulate polymer matrix; and
   (iii) an osteogenic protein-sequestering amount of autologous blood clot.

2. The composition of claim 1 wherein the osteogenic protein is a member of the BMP-family.

3. The composition of claim 2 wherein the osteogenic protein is BMP-2.

4. The composition of any one of claims 1 to 3 wherein the admixture is free from antifibrinolytic agents.

5. The composition of any one of claims 1 to 4 wherein the polymer matrix component is poly(lactic acid), poly(glycolic acid) or a copolymer of lactic acid and glycolic acid.

6. The composition of any one of claims 1 to 4 wherein the polymer matrix component is PLGA.

7. The composition of any one of claims 1 to 4 wherein the polymer matrix component is poly(orthoester), polyanhydride or a poly(propylene-co-fumarate) polymer.

8. The composition of any one of claims 1 to 3 wherein said polymeric matrix component comprises polymeric particles having a diameter of between about 150 and 850 µm and a porosity such that the surface area of the particles is between 0.01 and 4.0 m$^2$/g, wherein the polymer is poly(lactic acid), poly(glycolic acid) or a copolymer of lactic acid and glycolic acid.

9. A method for the manufacturing of a composition of any one of claims 1 to 8 comprising combining

   (i) an osteogenic protein;
   (ii) a porous particulate polymer matrix; and
   (iii) an osteogenic protein-sequestering amount of autologous blood clot to a pharmaceutically acceptable admixture.

## Patentansprüche

1. Zusammensetzung, die in der Lage ist, Knorpel- und/oder Knochenbildung in einem Patienten zu induzieren, umfassend ein pharmazeutisch verträgliches Gemisch aus

(i) einem osteogenen Protein;
(ii) einer porösen, aus Partikeln bestehenden Polymermatrix; und
(iii) einer osteogenes Protein sequestrierenden Menge von autologen Blutgerinnsein.

2. Zusammensetzung nach Anspruch 1, wobei das osteogene Protein ein Mitglied der BMP-Familie ist.

3. Zusammensetzung nach Anspruch 2, wobei das osteogene Protein BMP-2 ist.

4. Zusammensetzung nach einem der Ansprüche 1 bis 3, wobei das Gemisch frei von antifibrinolytischen Stoffen ist.

5. Zusammensetzung nach einem der Ansprüche 1 bis 4, wobei die Polymermatrix-Komponente Poly(milchsäure), Poly(glycolsäure) oder ein Copolymer aus Milchsäure und Glycolsäure ist.

6. Zusammensetzung nach einem der Ansprüche 1 bis 4, wobei die Polymermatrix-Komponente PLGA ist.

7. Zusammensetzung nach einem der Ansprüche 1 bis 4, wobei die Polymermatrix-Komponente ein Poly(orthoester), Polyanhydrid oder ein Poly(propylen-co-fumarat)-Polymer ist.

8. Zusammensetzung nach einem der Ansprüche 1 bis 3, wobei die Polymermatrix-Komponente Polymerpartikel umfaßt, die einen Durchmesser von etwa 150 bis 850 $\mu$m und eine Porösität besitzen, bei der die Oberfläche der Partikel zwischen 0,01 und 4,0 m$^2$/g ist, wobei das Polymer Poly(milchsäure), Poly(glycolsäure) oder ein Copolymer aus Milchsäure und Glycolsäure ist.

9. Verfahren zur Herstellung einer Zusammensetzung nach einem der Ansprüche 1 bis 8, umfassend das Kombinieren

(i) eines osteogenen Proteins;
(ii) einer porösen aus Partikeln bestehenden Polymermatrix; und
(iii) einer osteogenes Protein sequestrierenden Menge von autologen Blutgerinnseln
zu einem pharmazeutisch verträglichen Gemisch.

**Revendications**

1. Composition pouvant induire une formation cartilagineuse et/ou osseuse chez un patient comprenant un mélange pharmaceutiquement acceptable:

(i) d'une protéine ostéogénique;
(ii) d'une matrice de polymère particulaire poreuse; et
(iii) d'une quantité de caillot sanguin autologue séquestrante de protéine ostéogénique.

2. Composition suivant la revendication 1, dans laquelle la protéine ostéogénique est un membre de la famille des BMP.

3. Composition suivant la revendication 2, dans laquelle la protéine ostéogénique est de la BMP-2.

4. Composition suivant l'une quelconque des revendications 1 à 3, dans laquelle le mélange est exempt d'agents antifibrinolytiques.

5. Composition suivant l'une quelconque des revendications 1 à 4, dans laquelle le composant de matrice de polymère est du poly(acide lactique), poly(acide glycolique) ou un copolymère d'acide lactique et d'acide glycolique.

6. Composition suivant l'une quelconque des revendications 1 à 4, dans laquelle le composant de matrice de polymère est du PLGA.

7. Composition suivant l'une quelconque des revendications 1 à 4, dans laquelle le composant de matrice de polymère est un poly(orthoester), polyanhydride ou un polymère de poly(propylène-co-fumarate).

8. Composition suivant l'une quelconque des revendications 1 à 3, dans laquelle ledit composant de matrice polymérique comprend des particules polymériques ayant un diamètre entre environ 150 et 850 $\mu$m et une porosité telle que l'aire superficielle des particules se situe entre 0,01 et 4,0 m$^2$/g, dans laquelle le polymère est du poly(acide lactique), poly(acide glycolique) ou un copolymère d'acide lactique et d'acide glycolique.

9. Procédé de fabrication d'une composition suivant l'une quelconque des revendications 1 à 8, comprenant la combinaison:

(i) d'une protéine ostéogénique;
(ii) d'une matrice de polymère particulaire poreuse; et
(iii) d'une quantité de caillot sanguin autologue séquestrante de protéine ostéogénique,
à un mélange pharmaceutiquement acceptable.